# EUROPEAN PATENT SPECIFICATION

(11) **EP 1 596 748 B1**
(45) Date of publication and mention of the grant of the patent: **14.04.2010**
(21) Application number: 04710377.5
(22) Date of filing: 12.02.2004
(51) Int. Cl.: A61B 18/26

(54) **SURGICAL NEEDLE WITH LASER TARGET**
CHIRURGISCHE NADEL MIT LASER-TARGET
AIGUILLE CHIRURGICALE AVEC CIBLE LASER

(30) Priority: 12.02.2003 US 364880
(43) Date of publication of application: 23.11.2005
(73) Proprietor: Thyzel, Reinhardt, 90562 Heroldsberg (DE)
(72) Inventor: Thyzel, Reinhardt, 90562 Heroldsberg (DE)
(74) Representative: Schröer, Gernot H.
(86) International application number: PCT/EP2004/001303
(87) International publication number: WO 2004/071319

(56) References cited:
- DE-A- 19 907 012
- US-A- 5 041 121
- US-A- 5 324 282
- US-A- 5 906 611
- US-A- 5 957 914

## Description

### Background Of The Invention

In general, this invention relates to a laser powered surgical instrument which provides shockwaves for the ablation of tissue and more particularly to one that provides certain improvements over the surgical instruments shown in United States Patents No. 5,324,282 and No. 5,906,611.

The embodiment of the invention described is adapted to be used in eye surgery and particularly for cataract removal. However, the invention can be embodied in devices which are adapted to other surgical purposes.

The use of laser energy for eye surgery is well known. More particularly, employment of laser energy directed to a metal target to generate shockwaves which impinge on tissue to break up the tissue is known in the above referenced two patents.

The primary purpose of this surgical needle is for cataract surgery. The cataract tissue is held at the distal opening of the needle and is broken up by shockwaves that shatter the tissue on which the shockwaves impinge. These shockwaves are generated by application of laser pulses on a metal target located within the surgical needle adjacent to the opening of the needle at which the targeted tissue is positioned.

The surgical needle designs shown in the above two referenced patents have been successfully employed in surgery; the stepped target design of the '611 design being preferred.

However, there are operating features of known operating needles which it is desirable to improve and that would provide an enhanced surgical instrument.

More particularly, it is desirable that the device permits completion of the procedure with less operating time and use of less energy.

One advantage of a shorter operating time is that it provides less trauma and less risk to the patient. This shorter operating time provides overall enhanced patient function only when accompanied by a lower energy requirement and by an enhanced ability for the surgeon to navigate the needle with assurance of position.

For example, one feature that the surgeon refers to as "occlusion" is the ability of the distal opening to hold the tissue in place as it is being shattered by the shockwaves. In large part because it aids in providing a shorter operating time, it is an object of this invention to enhance the occlusion. When cataract tissue has been broken off by a pulse of energy, it is frequently too large to aspirate out of the small aspirating passageway in a needle. It is important that the tissue be held at the distal operating port of a needle so that a second or third pulse of ultrasonic energy will break down the tissue for ultimate aspiration.

A greater flow velocity of aspirating fluid enhances occlusion and thus helps to rapidly remove fractured tissue so that the ablating of tissue can proceed without obstruction and thus more rapidly. To achieve this greater velocity of aspirating fluid, it is desirable that there be as little turbulence as possible. Flow that is close to laminar flow will permit a more rapid flow of fluid and thus a more rapid removal of fractured or ablated tissue. A greater flow velocity will create a greater vacuum at the operating port that better holds the tissue and provides enhanced occlusion.

Thus it is an object of this invention to provide a structure and technique that provides enhanced occlusion and greater flow velocity as defined in claim 1.

It is important that the above objects be obtained in a device where additional structural features or complicated procedures are not required so that costs can be minimized and the surgeon will feel as comfortable as possible in using the device and the procedure associated therewith.

### Brief Description

In brief, one embodiment of this invention involves a 1.2 mm outside diameter needle having a distal operating port of about 0.6 mm to 0.8 mm. The operating port of the needle is at the distal end of the needle so that the surgeon's view of the operating area is minimally blocked. The target on which the laser energy impinges to generate the acoustic shockwaves is adjacent to the distal end of the target and positioned close to the operating port.

By having a shorter operating time, the target can be somewhat less massive than in the prior art. Thus the needle is designed with a blunt end at which the operating port is positioned so that the operating area can be more readily observed.

The 20.5 mm long needle is made of a unitary metal and thus without seams. This lack of seams reduces turbulence and permits flow closer to laminar flow or laminar-like flow than occurs in the prior designs thereby providing a flow velocity that better holds the tissue at the operating port and thus permits quicker removal of fractured tissue. This contributes to a lesser operating time.

In large part, because of a greater flow velocity, better occlusion of larger fragment tissue pieces is obtained at the operating port to assure immediate further shattering by subsequent shockwaves and then aspiration through the needle.

The target structure is best understood by reviewing the drawings. It is a structure somewhat different than the stepped target shown in Patent No. 5,906,611. The structure provides an optimum geometry to supply enough target material for the operation without requiring a more massive amount of target material. This less massive target makes possible an overall geometry which facilitates the use of this operating needle.

### On Terminology

### Flow Velocity, Flow Rate And Flow Volume

It is worthwhile keeping in mind a distinction between flow velocity, flow rate and flow volume. In large part because of the smaller operating port, the amount of fluid that is aspirated in a given time period (i.e., flow rate) is reduced over the prior art design. But because of a greater aspirating vacuum, the velocity of the fluid being aspirated into and through the needle is greater. This greater flow velocity helps to increase the occlusion characteristic of the operating port.

Further, even though the flow velocity in the needle is increased, a shorter operating time and lower flow rate means that the total volume of flow is reduced over the prior art. Thus, in this design, an increased flow velocity is coupled with a decreased flow rate and decreased flow volume.

### Smooth Surface.

The inner surface of the needle is referred to herein as smooth. This is the best geometric term for the surface characteristic that minimizes turbulence and minimizes friction. It provides laminar flow or close to laminar flow. In addition, it provides a reduced or minimal friction for the flow of fluid through the needle.

The smoother surface is created by fabricating the bore or lumen of the tube with a high speed (for example, 40,000 rpm) drill such as used in high quality watch making. This avoids having the small nicks or scratches on the inner surface that create or increase turbulence.

Having the needle as a unitary tube avoids the welding or other forming process that inevitably creates small bumps or discontinuities on the surface near the welding. These bumps or discontinuities create or increase turbulence.

The smooth bore surface required to achieve greater tissue retention calls for the combination of a unitary needle and a bore created by a high speed drill.

### Turbulence And Friction

Minimizing turbulence and friction is the key to obtaining a faster flow velocity which, in turn, creates the enhanced occlusion (retention) of particles at the distal operating port. The smooth surface of the bore in the needle, created by a high speed drilling technique, reduces turbulence and also reduces friction. This smooth bore surface makes it possible to maximize the velocity at which essentially laminar flow can be maintained. An attempt to increase the velocity to the point where turbulence occurs would in fact reduce the flow and reduce the ability to hold the particle being shattered at the distal port of the needle.

It is believed that this combination of maximized substantially laminar flow and minimized friction work together to provide an optimally high velocity and thus achieve enhanced tissue occlusion.

### Brief Description Of The Figures

FIG. 1 is a longitudinal sectional view through the first embodiment of the surgical needle of this invention.
FIG. 2 is an expanded sectional view of the distal end of the FIG. 1 instrument.
FIG. 3 is a side view of the distal end of the FIG. 1 instrument.
FIG. 4 is a perspective view of the distal end of the FIG. 1 instrument.
FIG. 5 is a partially cut away perspective view of the FIG. 1 distal end.
FIG. 6 is a longitudinal sectional view, similar to FIG. 1, of a second embodiment of this invention.

### Description Of The Preferred Embodiments

As shown in the FIGs., the surgical needle 10 has a unitary sidewall 12, an aspirating channel 14 and an optical fiber 16 adapted to convey laser energy.

The distal end of the needle 10 has a relatively blunt front surface 18 and an operating port 20. The target 22 is a complex surface comprising a primary surface 24 and a small hill 26. The central axis of the optical fiber 16 is in alignment with the hill portion 26 so that when the optical fiber supplies pulses of laser energy, the central component of those pulses will impinge on the hill 26 causing optical breakdown and the release of shockwaves that are then transmitted to the operating port 20. After the hill 26 has been ablated away, the main portion of the laser pulse energy will impinge on the main surface 24 providing further shockwaves. The hill 26 is not a necessary component of the design. It is an artifact of the process of drilling the port 20.

Because of the unitary sidewall 12, there are no ridges or bumps in the sidewall 12 which would induce turbulence. It is true that at the port 20, the suction of fluid into the aspirating channel 14 causes turbulence as does the front edge of the optical laser energy fiber 16. However, the smooth inner surface over ninety (90) percent of the needle which is proximal of the front end of the fiber 16 promotes a more laminar type of flow and thus permits a greater flow velocity than otherwise would be the case.

The bore that defines the channel 14 is created by a high speed (for example 40,000 rpm) drilling process such as is used in high quality watch making. This creates the channel 14 surface that has minimum nicks and scratches. The lack of ridges and bumps by avoiding a seaming process together with the minimized nicks and scratches provides the desired smooth channel 14 wall surface.

Irrigation is provided by a separate irrigating needle (not shown) of a type known in the art.

The circular port 20 is preferable over an elliptical port. The reason is that for a given maximum size particle to be aspirated, the circular port has a lesser cross-sectional area and thus provides a better trade-off of higher flow velocity and lower flow volume.

This combination of structural features provides a more optimum trade-off of functional features. The aspirating flow velocity is increased because of the less turbulent more laminar flow. This less turbulent more laminar flow arises because of a structural design which includes the unitary needle wall 12 having a smooth inside surface. The higher velocity flow due to a less turbulent aspirating flow, permits the use of a lesser quantity of fluid to provide an enhanced aspirating effect.

In large part, because of the greater flow velocity, pieces of tissue that have been ablated are more readily held at the operating port 20 to be shattered into smaller pieces that can be more readily aspirated by immediately successive shockwave pulses. This enhanced occlusion results in a shorter operating time.

In part as a consequence of the shorter operating time, the target 22 need not be as massive as in previous designs. Thus it can be designed to permit a needle at which the operating port 20 is at the distal end, rather than requiring a set back to accommodate a more massive target. In one embodiment, the thickness of the target 22 over the main target surface is 0.21 mm.

Having the operating port 20 at the distal end means that the surgeon's view of the operating zone where the tissue ablation occurs is minimally obstructed by the front surface of the needle. This provides the surgeon with a greater ability to navigate the needle with assurance and precision thereby contributing to the shorter operating time.

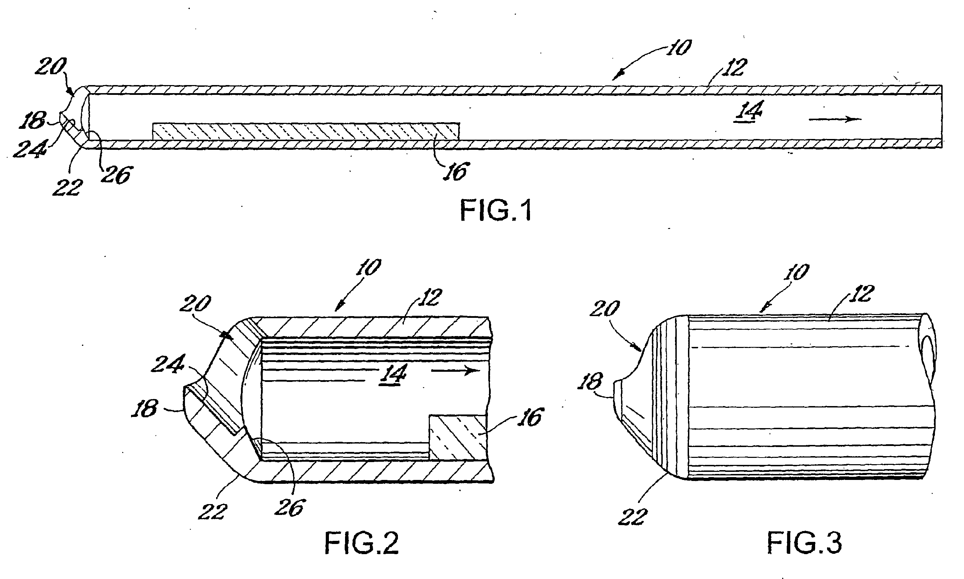

As may be seen in FIG. 2, the port 20 and target 24 have a geometric relationship to each other that contributes to the enhancement of this operating needle. In particular, a longitudinal plane perpendicular to the plane of FIG. 2 and extending through the axis of the wall 12 will show the port 20 to be on one side of that plane and the target 24 to be on the other side of that plane. In addition, the port 20 and the target 24 are in approximately the same radial zone at the end of the needle. Further, the plane of the target 24 is at approximately 45 degrees to the axis of the sidewall 12 and the central axis of the port 20 is at approximately 30 to 45 degrees to the central axis of the sidewall 12. This geometric combination assures that tissue ablation occurs at the front of the needle, is more readily visible to the surgeon, and that the shockwaves are directed onto the tissue to be fractured.

It is presently believed that a somewhat shorter laser pulse length (for example, four nano-seconds) may be advantageous in reducing the mass of target required, thereby contributing to most of the other parameters discussed above, while delivering adequate energy shockwaves to ablate tissue particularly where the tissue particles are better occluded at the port 20 so that they can be more quickly disposed of as smaller aspirated pieces by immediate successive shockwaves.

As may be seen in the above description, this combination of features positively reinforce one another to provide an optimum design. In a sense, many of these features are not so much trade-offs with one another as features which make it possible for the other feature to be effective.

For example, less turbulent flow due to the unitary, smooth sidewall 12 provides better occlusion which makes it possible to reduce the operating time which therefore allows for a less massive target 22 which in turn permits the tip design in which the port 20 is at the distal end so that the surgeon can better navigate the needle thereby reducing operating time that in turn permits the reduced mass of the target.

With respect to the target 22, the hill 26 is created by the forming technique that creates the port 20. The target could be a single surface at the angle shown.

The target 22 differs from the stepped target in Patent No. 5,906,611 in that the target surface 24, and the target surface of the hill 26, are at an angle (approximately 45 degrees) to the axis of the needle thereby providing a more direct path between the shockwaves generated in the mouth 20 than in the '611 patent design. It is believed that this more direct path makes a given energy shockwave more effective in breaking up tissue at the operating port 20.

In one preferred embodiment, the following dimensional arrangements exist. The needle 10 is 20.5 mm long, has an outside diameter of 1.2 mm, and an inside diameter of 0.9 mm and thus a very thin wall of 0.15 mm. The laser fiber is 0.34 mm in diameter. In that embodiment, the operating port 20 is circular and has a diameter of 0.6 mm to 0.8 mm and the axis of the port 20 is at approximately 45 degrees to the axis of the needle 10. The front end of the optical fiber 16 is 1.9 mm from the distal edge of the needle and approximately 1.3 mm from the beginning point of the operating surface provided by the hill 26. The curved distal tip of the needle 10 is approximately a spherical surface having a radius of curvature of 0.6 mm.

In that embodiment a known YAG laser provides laser energy at 1.064 nanometers in pulses having about 4 nano-second widths. The needle 10 including the target 22 is titanium.

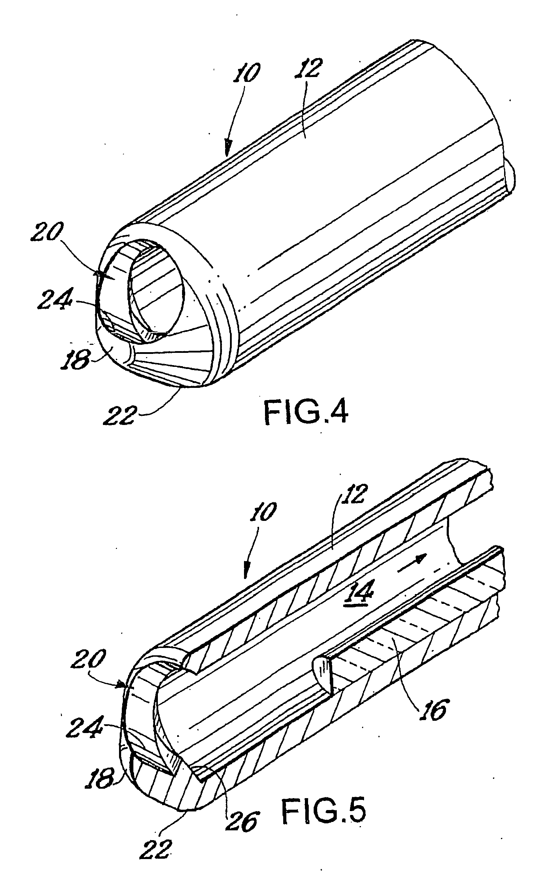

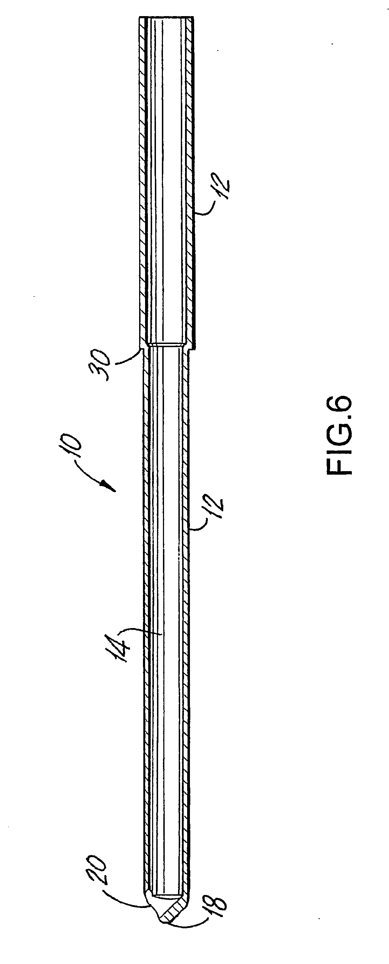

The FIG. 6 embodiment differs from the embodiment of FIGs. 1-5 in one major respect. It has a step 30 at about one third of the way from the proximal end of the needle 10. This step provides a 13 mm long distal sidewall section that is smaller and thinner than is the FIG. 1 embodiment. This enhances the surgeon's view and reduces trauma. The proximal sidewall section has the same O.D. as in the FIG. 1 embodiment so as to fit into a predetermined handle. This thinner sidewall section that is distal of the step 30 has one mm O.D. and a 0.8 mm I.D.

What has been found is that the channel diameter change at the step (a change from 0.8 mm to 0.9 mm) does not materially affect turbulence.

Thus, It should be understood that a smooth surface is consistent with having a minor step to increase the proximal diameter of the bore of the needle for the purpose of decreasing friction. A step that does not substantially increase turbulent flow is consistent with the definition herein of the smooth surface as one which is smooth enough to minimize turbulence and minimize friction.

While the foregoing description and drawings represent the presently preferred embodiments of the invention, it should be understood that those skilled in the art will be able to make changes and modifications to those embodiments without departing from the scope of the claims.

## Claims

1. A surgical needle (10) for fracturing tissue at an operating port (20) through the generation of shockwaves due to plasma formation from the optical breakdown of a target (22) on which laser pulses from a laser beam impinge, wherein
a) the operating port (20) is positioned at the distal end of the needle (10),
b) the target (22) has a wall mass which extends immediately proximal of the distal most portion of the operating port (20),
c) the needle (10) has a sidewall (12) and an aspirating channel (14), the inner surface of the sidewall being a surface of the aspirating channel (14),
***characterized in that***
d) the sidewall (12) is made of unitary metal without seams and has, thus, a smooth inner surface to provide for reduced turbulence and laminar flow or close to laminar flow and/or for a reduced or minimal friction for the flow of fluid in the aspirating channel (14).

2. The surgical needle (10) of claim 1 wherein said operating port (20) is substantially on the first side of a plane longitudinally bisecting the surgical needle (10), and said target (22) is substantially on the second side of said plane.

3. The surgical needle (10) of either of claims 1 or 2 wherein said operating port (20) is substantially circular.

4. The surgical needle (10) of any of claims 1 through 3 wherein said target (22) has a target surface which is a plane at approximately 45 degrees to the axis of said aspirating channel (14).

5. The surgical needle (10) of any of claims 1 through 4 having an optical fiber for conveying the laser pulses, wherein the sole turbulence inducing structure in the aspirating channel (14) proximal of said operating port (20) and said target (22) is the optical fiber.

6. The surgical needle (10) of any of claims 1 through 5 wherein said target (22) and said operating port (20) extend over approximately the same longitudinal distance of the surgical needle (10).

7. The surgical needle (10) of any of claims 1 through 6 wherein said operating port (20) has a central axis and said needle (10) has a central axis, said central axis of said port (20) and said central axis of said needle (10) being at approximately 30 to 45 degrees to one another.

8. The surgical needle (10) according to any of the preceding claims, wherein the needle (10) including the target (22) is made of titanium.

9. The surgical needle (10) according to any of the preceding claims, wherein the aspirating channel (14) is defined by a bote.

10. The surgical needle (10) according to claim 9, wherein the bore is created by a high speed drilling process, preferably at 40,000 rpm.

## Patentansprüche

1. Chirurgische Nadel (10) zum Aufbrechen von Gewebe an einer Arbeitsdurchgangsöffnung (20) durch Erzeugung von Stoßwellen infolge einer Plasmabildung aus dem optischen Durchbruch eines Ziels (22), auf das Laserimpulse aus einem Laserstrahl auftreffen, bei welcher
a) die Arbeitsdurchgangsöffnung (20) am distalen Ende der Nadel (10) positioniert ist,
b) das Ziel (22) eine Wandungsmasse aufweist, die sich unmittelbar proximal vom am weitesten in distaler Richtung liegenden Abschnitt der Arbeitsdurchgangsöffnung (20) erstreckt,
c) die Nadel (10) eine Seitenwandung (12) und einen Ansaugkanal (14) aufweist, wobei die Innenfläche der Seitenwandung eine Oberfläche des Ansaugkanals (14) darstellt,
**dadurch gekennzeichnet, dass**
d) die Seitenwandung (12) aus einem einheitlichen Metall ohne Nahtstellen besteht und somit eine glatte Innenfläche aufweist, so dass für verringerte Turbulenz und für Fließströmung bzw. nahezu Fließströmung und/oder für eine verringerte bzw. minimale Reibung für den Strömungsmittelfluss im Ansaugkanal (14) gesorgt ist.

2. Chirurgische Nadel (10) nach Anspruch 1, bei welcher sich die Arbeitsdurchgangsöffnung (20) im Wesentlichen auf der ersten Seite einer Ebene befindet, die in Längsrichtung die chirurgische Nadel (10) halbiert, und das Ziel (22) sich im Wesentlichen auf der zweiten Seite der Ebene befindet.

3. Chirurgische Nadel (10) nach Anspruch 1 oder 2, bei welcher die Arbeitsdurchgangsöffnung (20) im Wesentlichen kreisförmig ist.

4. Chirurgische Nadel (10) nach einem der Ansprüche 1 bis 3, bei welcher das Ziel (22) eine Zielfläche aufweist, die eine Ebene unter etwa 45 Grad zur Achse des Ansaugkanals (14) darstellt.

5. Chirurgische Nadel (10) nach einem der Ansprüche 1 bis 4 mit einer Lichtleitfaser zur Weiterleitung der Laserimpulse, bei welcher die einzige Struktur im Ansaugkanal (14), die proximal relativ zur Arbeitsdurchgangsöffnung (20) und dem Ziel (22) eine Turbulenz herbeiführt, die Lichtleitfaser ist.

6. Chirurgische Nadel (10) nach einem der Ansprüche 1 bis 5, bei welcher sich das Ziel (22) und die Arbeitsdurchgangsöffnung (20) über etwa den gleichen Abstand in Längsrichtung der chirurgischen Nadel (10) erstrecken.

7. Chirurgische Nadel (10) nach einem der Ansprüche 1 bis 6, bei welcher die Arbeitsdurchgangsöffnung (20) eine Mittelachse aufweiset und die Nadel (10) eine Mittelachse besitzt, wobei die Mittelachse der Durchgangsöffnung (20( und die Mittelachse der Nadel (10) unter einem Winkel von etwa 30 bis 45 Grad relativ zu einander stehen.

8. Chirurgische Nadel (10) nach einem der vorhergehenden Ansprüche, bei welcher die Nadel (10), die das Ziel (22) enthält, aus Titan gefertigt ist.

9. Chirurgische Nadel (10) nach einem der vorhergehenden Ansprüche, bei welcher der Ansaugkanal (14) von einer Bohrung definiert wird.

10. Chirurgische Nadel (10) nach Anspruch 9, bei welcher die Bohrung mittels eines Hochgeschwindigkeits-Bohrvorgangs, vorzugsweise mit 40.000 UpM, gebildet wird.

## Revendications

1. Aiguille chirurgicale (10) pour fracturer le tissu à un orifice d'opération (20) par la génération d'ondes de choc dues à la formation de plasma à partir de la rupture optique d'une cible (22) sur laquelle des impulsions laser d'un faisceau laser sont incidentes, où
a) l'orifice d'opération (20) est positionné à l'extrémité distale de l'aiguille (10),
b) la cible (22) possède une masse de paroi qui s'étend directement proximalement de la portion la plus distale de l'orifice d'opération (20),
c) l'aiguille (10) possède une paroi latérale (12) et un canal d'aspiration (14), la surface interne de la paroi latérale étant une surface du canal d'aspiration (14),
**caractérisée en ce que**
d) la paroi latérale (12) est réalisée en métal unitaire sans soudures et possède ainsi une surface intérieure lisse pour réaliser une turbulence réduite et un écoulement laminaire ou proche d'un écoulement laminaire et/ou pour une friction réduite ou minimale pour l'écoulement du fluide dans le canal d'aspiration (14).

2. Aiguille chirurgicale (10) selon la revendication 1, dans laquelle ledit orifice d'opération (20) est sensiblement sur le premier côté d'un plan bissectant longitudinalement l'aiguille chirurgicale (10), et ladite cible (22) se trouve sensiblement sur le second côté dudit plan.

3. Aiguille chirurgicale (10) selon l'une quelconque des revendications 1 ou 2, dans laquelle ledit orifice d'opération (20) est sensiblement circulaire.

4. Aiguille chirurgicale (10) selon l'une quelconque des revendications 1 à 3, dans laquelle ladite cible (22) possède une surface cible qui est un plan approximativement à 45 degrés à l'axe dudit canal d'aspiration (14).

5. Aiguille chirurgicale (10) selon l'une quelconque des revendications 1 à 4, ayant une fibre optique pour convoyer les impulsions laser, dans laquelle l'unique structure induisant des turbulences dans le canal d'aspiration (14) proximal dudit orifice d'opération (20) et de ladite cible (22) est la fibre optique.

6. Aiguille chirurgicale (10) selon l'une quelconque des revendications 1 à 5, dans laquelle ladite cible (22) et ledit orifice d'opération (20) s'étendent sur environ la même distance longitudinale de l'aiguille chirurgicale (10).

7. Aiguille chirurgicale (10) selon l'une quelconque des revendications 1 à 6, dans laquelle ledit orifice d'opération (20) possède un axe central, et ladite aiguille (10) possède un axe central, ledit axe central dudit orifice (20) et ledit axe central de ladite aiguille (10) étant environ à 30 jusqu'à 45 degrés l'un à l'autre.

8. Aiguille chirurgicale (10) selon l'une quelconque des revendications précédentes, dans laquelle l'aiguille (10) incluant la cible (22) est réalisée en titane.

9. Aiguille chirurgicale (10) selon l'une quelconque des revendications précédentes, dans laquelle le canal d'aspiration (14) est défini par un perçage.

10. Aiguille chirurgicale (10) selon la revendication 9, dans laquelle le perçage est crée par un processus de perçage à haute vitesse, de préférence à 40 000 tr/min.
